# EUROPÄISCHE PATENTSCHRIFT

(11) **EP 2 907 497 B1**
(45) Veröffentlichungstag und Bekanntmachung des Hinweises auf die Patenterteilung: **03.07.2019**
(21) Anmeldenummer: 15154608.2
(22) Anmeldetag: 11.02.2015
(51) Int. Cl.: A61K 8/39, A61K 8/44, A61Q 17/04, A61K 8/06

(54) **Kosmetische Emulgatorkombination**
Cosmetic emulsifier combination
Combinaison d'émulsifiants cosmétiques

(30) Priorität: 18.02.2014 DE 102014202956
(43) Veröffentlichungstag der Anmeldung: 19.08.2015
(73) Patentinhaber: Beiersdorf AG, 20253 Hamburg (DE)
(72) Erfinder: Brinkmann, Sina, 21698 Harsefeld (DE); Weinert, Katrin, 22763 Hamburg (DE); Bleckmann, Andreas, 22926 Ahrensburg (DE); Schade, Tatjana, 25866 Mildstedt/Rosendahl (DE)

(56) Entgegenhaltungen:
- EP-A1- 1 857 091
- WO-A2-2009/018974
- WO-A2-2013/092186
- DE-A1-102007 038 413
- DE-A1-102011 077 018
- JP-B2- 4 780 836
- Anonymous: "Face Age Control SPF 15", GNPD Id 2027397, 1. März 2013 (2013-03-01), XP055204982, Gefunden im Internet: URL:http://www.gnpd.com/sinatra/recordpage /2027397/from_search/pdPTCzpy1w/ [gefunden am 2015-07-28]

## Beschreibung

Die vorliegende Erfindung betrifft eine kosmetische Emulsion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile. Emulgatoren mit einem besonders hohen HLB-Wert (größer 13) können auch als waschaktive Tenside eingesetzt werden.

Um stark unpolare Öle und hydrophobe Feststoffe, beispielsweise partikuläre organische Lichtschutzfilter, stabil in Emulsionen einarbeiten zu können, darf der Gesamt-HLB-Wert des Emulgatorsystems nicht zu hoch gewählt werden. Mit steigendem Gesamt-HLB werden die Emulsionen sonst zunehmend instabil (insbesondere bei thermischen und mechanischen Belastungen und mit zunehmender Lagerdauer). Die Emulsionen neigen dann zur Ölabscheidung, Phasentrennung und ggf. auch zu Ausfällungen von Feststoffen.

Andererseits sind Zubereitungen mit hohem Gesamt-HLB-Wert sensorisch besonders attraktiv, da sie besonders wenig fettig auf der Haut wirken.

Es war daher die Aufgabe der vorliegenden Erfindung, ein besonders stabiles und sensorisch attraktives Emulgatorsystem zu entwickeln, in welches auch unpolare Öle und partikuläre organische Lichtschutzfilter stabil eingearbeitet werden können.

Nicht zuletzt war es die Aufgabe der vorliegenden Erfindung ein besonders einfaches und kostengünstiges Emulgatorsystem (sowie ein Herstellungsverfahren desselben) zu entwickeln.

Überraschend gelöst werden konnte die Aufgabe durch eine kosmetische Zubereitung gemäß Anspruch 1.

Zwar kennt der Fachmann die EP 0 766 959, WO 2009/0189741 und WO 2009/063392, sowie die DE 10 2011 077018, WO 2013/092186, EP 1 857 091, JP 4 780836, WO 2009/018974, DE 10 2007 038413 sowie den Datenbank-Eintrag der Mintel GNPD-Datenbank "Face Age Control SPF 15 mit der Record ID 2027397 doch konnten diese Schriften nicht den Weg zur vorliegenden Erfindung weisen.

Es ist erfindungsgemäß bevorzugt, wenn die Zubereitung als Polyglyceryl Monoalkylester Polyglyceryl-10 Stearate, Polyglyceryl-10 Laurate und/oder Polyglyceryl-10 Caprate eingesetzt werden. Ganz besonders bevorzugt ist dabei das Polyglycerin-10 Laurate.

Durch die erfindungsgemäße Kombination kann die Einsatzkonzentration an Emulgatoren in den Zubereitungen insgesamt überraschend deutlich reduziert werden. Dieser Aspekt ist besonders für Zubereitungen von Bedeutung, die für Menschen mit empfindlicher Haut oder Mallorca-Akne entwickelt werden.

Es ist erfindungsgemäß vorteilhaft, wenn die erfindungsgemäße Zubereitung in Form einer O/W-Emulsion vorliegt.

Erfindungsgemäß vorteilhaft enthält die erfindungsgemäße Zubereitung Natriumstearylglutamat in einer Konzentration von 0,05 bis 2 Gewichts-% und erfindungsgemäß bevorzugt in einer Konzentration von 0,1 bis 1 Gewichts-%, jeweils bezogen auf das Gesamtgewicht der Zubereitung.
Das erfindungsgemäße Natriumstearylglutamat kann beispielsweise unter dem Namen Eumulgin SG bei der Firma BASF erworben werden.

Erfindungsgemäß vorteilhaft enthält die erfindungsgemäße Zubereitung Polyglyceryl Monoalkylester in einer Gesamtkonzentration von 0,05 bis 1 Gewichts-% und erfindungsgemäß bevorzugt in einer Gesamtkonzentration von 0,075 bis 0,5 Gewichts-%, jeweils bezogen auf das Gesamtgewicht der Zubereitung.

Es ist erfindungsgemäß vorteilhaft, wenn die Zubereitung den UV-Filter 2,4,6-Tris-(biphenyl)-1,3,5-triazin enthält.

2,4,6-Tris-(biphenyl)-1,3,5-triazin wird dabei erfindungsgemäß vorteilhaft in einer Menge von 0,2 bis 10 Gewichts-% und bevorzugt in einer Menge von 0,5 bis 5 Gewichts-%, bezogen auf das Gesamtgewicht der Zubereitung eingesetzt.

Es ist erfindungsgemäß vorteilhaft, wenn die Zubereitung den UV-Filter 2,2'-Methylen-bis-(6-(2H-benzotriazol-2-yl)-4-(1,1,3,3-tetramethylbutyl)-phenol) enthält.

2,2'-Methylen-bis-(6-(2H-benzotriazol-2-yl)-4-(1,1,3,3-tetramethylbutyl)-phenol) wird dabei erfindungsgemäß vorteilhaft in einer Menge von 0,2 bis 10 Gewichts-% und bevorzugt in einer Menge von 0,5 bis 5 Gewichts-%, bezogen auf das Gesamtgewicht der Zubereitung eingesetzt.

Es ist erfindungsgemäß vorteilhaft, wenn die Zubereitung das Hydroxyphenyl Benzophenon (Verbindung 1) mit der folgenden Struktur enthält:

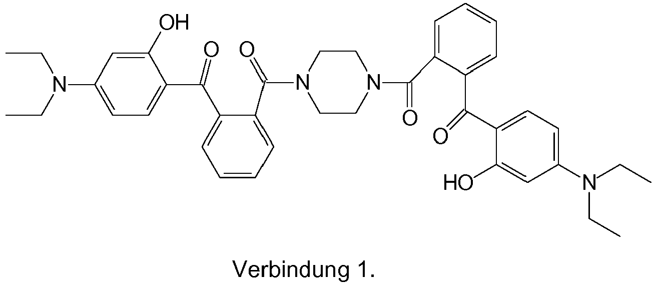

Dieses Hydroxyphenyl Benzophenon wird dabei erfindungsgemäß vorteilhaft in einer Menge von 0,2 bis 10 Gewichts-% und bevorzugt in einer Menge von 0,5 bis 5 Gewichts-%, bezogen auf das Gesamtgewicht der Zubereitung eingesetzt.

Erfindungsgemäß vorteilhafte Ausführungsformen der vorliegenden Erfindung sind dadurch gekennzeichnet, dass die Zubereitung UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 4-Dicyanomethylen-2,6-dimethyl-1,4-dihydropyridin-N-(ethyloxysulfatestersalze), Titandioxid; Zinkoxid; Polysiloxancopolymer mit einer statistischen Verteilung gemäß der Formel:

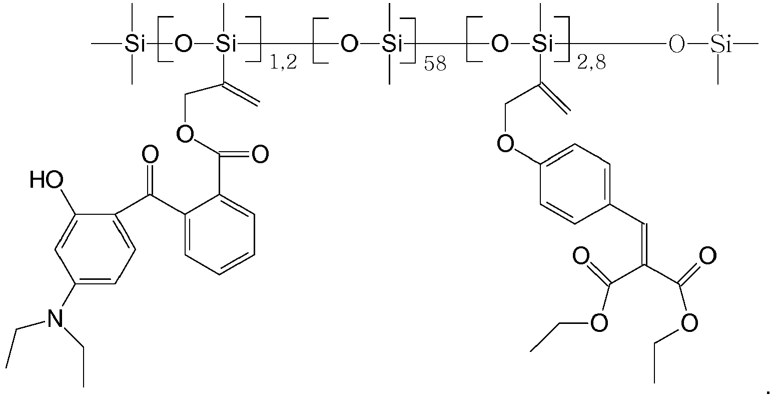

Diese UV-Filter können in Einzelkonzentrationen von 0,1 bis 30 Gewichts-%, bezogen auf das Gesamtgewicht der Zubereitung, in dieser enthalten sein.

Es ist erfindungsgemäß vorteilhaft, wenn die erfindungsgemäße Zubereitung frei ist von p-Methylbenzylidencampher.

Erfindungsgemäß vorteilhafte Ausführungsformen der vorliegenden Erfindung sind dadurch gekennzeichnet, dass die Zubereitung Sucrosepolystearat enthält.

Sucrosepolystearat kann beispielsweise unter dem Namen Emulgade Sucro bei der Firma BASF erworben werden.

Sucrosepolystearat wird erfindungsgemäß vorteilhaft in einer Menge von 0,1 bis 6 Gewichts-% und bevorzugt in einer Menge von 0,2 bis 4 Gewichts-%, bezogen auf das Gesamtgewicht der Zubereitung eingesetzt.

Es ist vorteilhaft im Sinne der vorliegenden Erfindung, wenn die erfindungsgemäße Zubereitung hydriertes Polyisobuten enthält.

Hydriertes Polyisobuten wird erfindungsgemäß vorteilhaft in einer Menge von 0,05 bis 2 Gewichts-% und bevorzugt in einer Menge von 0,1 bis 1 Gewichts-%, bezogen auf das Gesamtgewicht der Zubereitung eingesetzt.

Es ist erfindungsgemäß vorteilhaft, wenn die Zubereitung vernetztes Polymer aus Vinylpyrrolidon und Acrylsäure enthält.

Dabei ist es erfindungsgemäß bevorzugt, wenn als vernetztes Polymer aus Vinylpyrrolidon und Acrylsäure die Verbindung mit der CAS No: 527685-31-0 eingesetzt wird.

Das vernetzte Polymer aus Vinylpyrrolidon und Acrylsäure wird erfindungsgemäß vorteilhaft in einer Menge von 0,05 bis 4 Gewichts-% und bevorzugt in einer Menge von 0,1 bis 3 Gewichts-%, bezogen auf das Gesamtgewicht der Zubereitung eingesetzt.

Erfindungsgemäß vorteilhafte Ausführungsformen der vorliegenden Erfindung sind dadurch gekennzeichnet, dass die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Glycyrrhetinsäure und/oder Licochalcon A enthält.

Derartige Wirkstoffe sind erfindungsgemäß vorteilhaft in einer Konzentration von 0,01 bis 10 Gewichts-% und bevorzugt in einer Konzentration von 0,5 bis 5 Gewichts-%, bezogen auf das Gesamtgewicht der Zubereitung, in dieser enthalten.

Es ist vorteilhaft im Sinne der vorliegenden Erfindung, wenn die Zubereitung ein oder mehrere Diole gewählt aus der Gruppe der Verbindungen Heptan-1,2-diol, Nonan-1,2-diol und Ethylhexylglycerin enthält.

Derartige Diole können erfindungsgemäß vorteilhaft in einer Konzentration von 0,1 bis 20 Gewichts-%, bezogen auf das Gesamtgewicht der Zubereitung, in dieser enthalten sein.

Derartige Diole können erfindungsgemäß bevorzugt in einer Konzentration von 0,5 bis 10 Gewichts-%, bezogen auf das Gesamtgewicht der Zubereitung, in dieser enthalten sein.

Es ist erfindungsgemäß besonders vorteilhaft, wenn die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Dabei ist ein Alkandiol-Gehalt dieser Alkandiole von 0,5 bis 5 Gewichts-%, bezogen auf das Gesamtgewicht der Zubereitung erfindungsgemäß vorteilhaft.

Es ist erfindungsgemäß vorteilhaft, wenn die Zubereitung Phenoxyethanol enthält. Dabei ist ein Phenoxyethanol-Gehalt von 0,001 bis 1 Gewichts-%, bezogen auf das Gesamtgewicht der Zubereitung erfindungsgemäß vorteilhaft.

Erfindungsgemäß vorteilhaft ist die erfindungsgemäße Zubereitung frei von Parabenen (Methyl-, Ethyl-, Propyl-, Butylparaben).

Erfindungsgemäß bevorzugt ist die erfindungsgemäße Zubereitung frei von Polyethylenglycolen, d.h. PEG-frei.

In einer erfindungsgemäß vorteilhaften Ausführungsform ist die erfindungsgemäße Zubereitung sprühbar. Sie kann auch zur Tränkung von Substraten (z.B.: Tüchern, Pads etc.) verwendet werden. Für gewöhnlich liegt die erfindungsgemäße Zubereitung jedoch als Creme oder Lotion vor.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 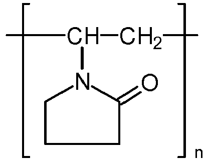 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der Firma Ashland Speciality Ingredients erhältlich sind.

Ebenfalls vorteilhaft sind weitere polymere Filmbildner, wie beispielsweise Natriumpolystryrensulfonat, welches unter der Handelsbezeichnung Flexan II bei der Firma Akzo Nobel erhältlich ist, und/oder Polyisobuten, erhältlich bei Evonik Industries AG unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen.

Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der BASF erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. HallStar erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß bevorzugt, wenn die erfindungsgemäße Zubereitung keine Fettalkohole enthält.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Erfindungsgemäß ist auch das Verfahren zur Herstellung der kosmetischen Zubereitung, welches dadurch gekennzeichnet ist, dass der Polyglyceryl Monoalkylester (bevorzugt Polyglyceryl-10 Monoalkylester, besonders bevorzugt Polyglyceryl-10 Stearate, Polyglyceryl-10 Laurate und/oder Polyglyceryl-10 Caprate) zunächst zusammen mit dem organischen UV-Filter aus der Gruppe 2,4,6-Tris-(biphenyl)-1,3,5-triazin; 2,2'-Methylen-bis-(6-(2H-benzotriazol-2-yl)-4-(1,1,3,3-tetramethylbutyl)-phenol) und/oder dem Hydroxyphenyl Benzophenon der Verbindung 1 zu einer wässrigen Vor-Dispersion vereinigt wird, die nach der Homogenisation in die abgekühlte fertige Emulsion (welche die restlichen Bestandteile der Zubereitung enthält) unter Rühren zugegeben wird.
Die Stabilität der kosmetischen Emulsionen, die nach diesem Verfahren hergestellt wird, ist besonders gut.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Emulsion** | **1** | **2** | **3** | **4** | **5** |
|---|---|---|---|---|---|
| Natrium Stearoylglutamat | 0,2 | 0,1 | 0,2 | 0,4 | 0,1 |
| Polyglyceryl Monoalkylester | 0,15 | 0,3 | 0,3 | 0,6 | 0,45 |
| Copolymer aus Vinylpyrrolidon und Acrylsäure | 0,5 | | | 1 | |
| Acrylat/C10-30 Alkyl Acrylat Crosspolymer | | 0,4 | 0,2 | | |
| Carbomer | | | 0,2 | | |
| Natrium Polyacrylate | | | | | 0,6 |
| C₁₂₋₁₅Alkyl Benzoat | 5 | | 8 | | 3 |
| Butylenglycol Dicaprylat/Dicaprat | 5 | | | 4 | |
| Phenethylbenzoat | | | | 4 | |
| Octyldodekanol | | 3 | | 1 | |
| Dicaprylylcarbonat | | 4 | | | 4 |
| Caprylic/Capric Triglycerid | | | 2 | | |
| Isodecyl Neopentanoat | | | 2 | | |
| Cyclomethicon | 2 | | | 3 | 2 |
| Dimethicon | | 2 | | 2,5 | |
| Glycerin | 2 | 5 | 4 | 3 | 2,5 |
| 2,2'-Methylen-bis-(6-(2H-benzotriazol-2-yl)-4-(1,1,3,3-tetramethylbutyl)-phenol) | 1 | | | | 1 |
| 2,4,6-Tris-(biphenyl)-1,3,5-triazin | | 2 | | 4 | 2 |
| Verbindung 1 | | | 2 | | |
| Bis-Ethylhexyloxyphenol Methoxyphenyl Triazin | | 3 | 1 | | |
| Ethylhexyltriazin | 2 | | | | |
| Butyl Methoxydibenzoylmethan | | | | 4 | 4 |
| Diethylamino Hydroxybenzoyl Hexyl Benzoat | | | 3 | | |
| Ethylhexyl Methoxycinnamat | | | | | 6 |
| Octylsalicylat | | | 5 | | |
| Octocrylen | | | | | 6 |
| Phenylbenzimidazol Sulfonsäure | | | | 2 | |
| Titandioxid | 3 | | | | |
| Ethanol | | | 4 | | 3 |
| Tocopherolacetat | 0,1 | 0,2 | | 0,3 | |
| Parfüm, Konservierungsmittel | q.s. | q.s. | q.s. | q.s. | q.s. |
| Farbstoffe, Neutralisationsmittel etc | q.s. | q.s. | q.s. | q.s. | q.s. |
| Wasser | ad 100,0 | ad 100,0 | ad 100,0 | ad 100,0 | ad 100,0 |

| **Emulsion** | **6** | **7** | **8** | **9** | **10** |
|---|---|---|---|---|---|
| Natrium Stearoylglutamat | 0,2 | 0,1 | 0,2 | 0,1 | 0,1 |
| Polyglyceryl Monoalkylester | 0,3 | 0,3 | 0,3 | 0,6 | 0,45 |
| Sucrose Polystearat + hydriertes Polyisobuten | 1 | 0,5 | 1 | 1 | 1,3 |
| Cetearylalkohol | | | | 1 | |
| Myristyl Myristat | | | | 2 | |
| Copolymer aus Vinylpyrrolidon und Acrylsäure | | | 0,6 | | 1,5 |
| Acrylat/C10-30 Alkyl Acrylat Crosspolymer | 0,2 | | | | |
| Carbomer | | | | | |
| Natrium Polyacrylate | 0,4 | | | 0,5 | |
| C₁₂₋₁₅Alkyl Benzoat | 4 | | | | |
| Butylenglycol Dicaprylat/Dicaprat | 3 | | | 5 | |
| Diisopropylsebacat | | | 4 | | 3 |
| Octyldodekanol | | | | 3 | |
| Dicaprylylcarbonat | | | | 3 | |
| Caprylic/Capric Triglycerid | | | | | 2 |
| Isodecyl Neopentanoat | | | | | |
| Cyclomethicon | | | 4 | | 5 |
| Dimethicon | | | | | |
| Glycerin | 8 | 3 | 5 | 1 | 2 |
| 2,2'-Methylen-bis-(6-(2H-benzotriazol-2-yl)-4-(1,1,3,3-tetramethylbutyl)-phenol) | 2 | | | | 1 |
| 2,4,6-Tris-(biphenyl)-1,3,5-triazin | | 2 | 2 | | 1 |
| Verbindung 1 | | | | 4 | |
| Bis-Ethylhexyloxyphenol Methoxyphenyl Triazin | 2 | | 2 | | |
| Ethylhexyltriazin | | | | 2 | |
| Butyl Methoxydibenzoylmethan | 3 | | | | |
| Diethylamino Hydroxybenzoyl Hexyl Benzoat | | | 5 | | |
| Polysilicon-15 | | | | | |
| Ethylhexyl Methoxycinnamat | 10 | | | | |
| Octylsalicylat | | | | 5 | |
| Homosalat | | | | | |
| Octocrylen | | | 5 | | |
| Phenylbenzimidazol Sulfonsäure | | | | 1 | |
| Ethanol | | | 2 | 2 | |
| Tocopherolacetat | | | 0,1 | | |
| Parfüm, Konservierungsmittel | q.s. | q.s. | q.s. | q.s. | q.s. |
| Farbstoffe, Neutralisationsmittel etc | q.s. | q.s. | q.s. | q.s. | q.s. |
| Wasser | ad 100,0 | ad 100,0 | ad 100,0 | ad 100,0 | ad 100,0 |

| **Emulsion** | **11** | **12** | **13** | **14** | **15** |
|---|---|---|---|---|---|
| Natrium Stearoylglutamat | 0,1 | 0,1 | 0,2 | 0,2 | 0,3 |
| Polyglyceryl Monoalkylester | 0,6 | 0,6 | 0,6 | 0,075 | 0,15 |
| Sucrose Polystearat | | | 0,8 | 1 | |
| Cetylalkohol | | | 3 | | |
| C18-38 Säuretriglycerid | | 1 | | | |
| Copolymer aus Vinylpyrrolidon und Acrylsäure | 0,2 | 1 | | | 0,5 |
| Acrylat/C10-30 Alkyl Acrylat Crosspolymer | 0,2 | | | 0,3 | |
| Carbomer | 0,2 | | | | |
| Natrium Polyacrylat | | | | 0,3 | |
| Vaseline | 2 | | | | |
| C₁₂₋₁₅Alkyl Benzoat | 8 | | | | |
| Butylenglycol Dicaprylat/Dicaprat | 5 | | | | 3 |
| 2-Propylheptyloctanoat | | 5 | | 3 | |
| Octyldodekanol | | | 2 | | |
| Dicaprylylcarbonat | 1 | 5 | | 2 | |
| Caprylic/Capric Triglycerid | | | 2 | | |
| Dibutyladipat | | | 2 | | 2 |
| Cyclomethicon | 3 | | | 2 | |
| Dimethicon | | 3 | | | |
| Glycerin | 2 | 3 | 1 | 4 | 5 |
| 2,2'-Methylen-bis-(6-(2H-benzotriazol-2-yl)-4-(1,1,3,3-tetramethylbutyl)-phenol) | | | 4 | | 1 |
| 2,4,6-Tris-(biphenyl)-1,3,5-triazin | 2 | 4 | | 0,5 | |
| Verbindung 1 | 2 | | | | |
| Bis-Ethylhexyloxyphenol Methoxyphenyl Triazin | | | | 1 | |
| Ethylhexyltriazin | 2 | | | 1 | |
| Butyl Methoxydibenzoylmethan | | 2 | | | |
| Diethylamino Hydroxybenzoyl Hexyl Benzoat | | | | 2 | |
| Polysilicon-15 | | 2 | | | |
| Ethylhexyl Methoxycinnamat | | | | | 5 |
| Octylsalicylat | | | 5 | | |
| Dioctylbutylamidotriazin | 1 | | | | |
| Phenylbenzimidazol Sulfonsäure | | 2 | | | |
| Dinatrium Phenylen-1,4-bis-(2-benzimidazyl)-3,3'-5,5'-tetrasulfonsäuresalz | | | 0,5 | | |
| Ethanol | 1 | 2 | | 2 | |
| Coenzym Q10 | 0,1 | 0,05 | | 0,05 | |
| Folsäure | | | 0,1 | 0,05 | |
| Parfüm, Konservierungsmittel | q.s. | q.s. | q.s. | q.s. | q.s. |
| Farbstoffe, Neutralisationsmittel etc | q.s. | q.s. | q.s. | q.s. | q.s. |
| Wasser | ad 100,0 | ad 100,0 | ad 100,0 | ad 100,0 | ad 100,0 |

## Patentansprüche

1. Kosmetische Zubereitung enthaltend eine Kombination aus
a) Natriumstearylglutamat,
b) Polyglyceryl Monoalkylester,
wobei die Zubereitung 2,4,6-Tris-(biphenyl)-1,3,5-triazin, 2,2'-Methylen-bis-(6-(2H-benzotriazol-2-yl)-4-(1,1,3,3-tetramethylbutyl)-phenol) und/oder das Hydroxyphenyl Benzophenon mit der folgenden Struktur (Verbindung 1) enthält: 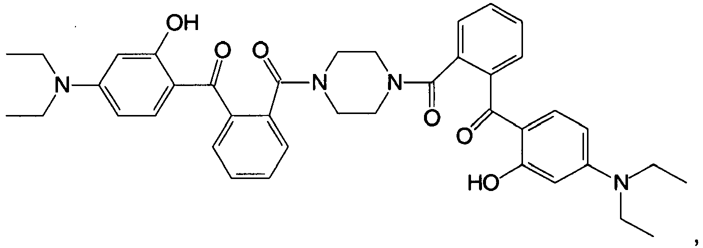 **dadurch gekennzeichnet, dass** die Zubereitung einen Polyglyceryl-10 Monoalkylester als Polyglyceryl Monoalkylester enthält.

2. Kosmetische Zubereitung nach Anspruch 1, **dadurch gekennzeichnet, dass** die Zubereitung als Polyglyceryl Monoalkylester Polyglyceryl-10 Stearate, Polyglyceryl-10 Laurate und/oder Polyglyceryl-10 Caprate eingesetzt werden.

3. Kosmetische Zubereitung nach einem der vorhergehenden Ansprüche, **dadurch gekennzeichnet, dass** die Zubereitung UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-di-phenylacrylat; Dimethicodiethylbenzalmalonat; 2-Ethylhexyl-2-cyano-3,3-diphenyl-acrylat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 4-Dicyanomethylen-2,6-dimethyl-1,4-dihydropyridin-N-(ethyloxysulfatestersalze), Titandioxid; Zinkoxid; Polysiloxancopolymer mit einer statistischen Verteilung gemäß der Formel:

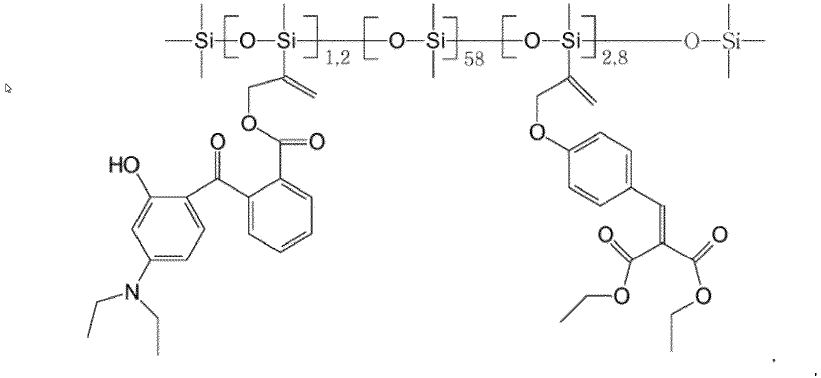

4. Kosmetische Zubereitung nach einem der vorhergehenden Ansprüche, **dadurch gekennzeichnet, dass** die Zubereitung Sucrosepolystearat enthält.

5. Kosmetische Zubereitung nach einem der vorhergehenden Ansprüche, **dadurch gekennzeichnet, dass** die Zubereitung vernetztes Polymer aus Vinylpyrrolidon und Acrylsäure enthält.

6. Kosmetische Zubereitung nach einem der vorhergehenden Ansprüche, **dadurch gekennzeichnet, dass** die Zubereitung hydriertes Polyisobuten enthält.

7. Kosmetische Zubereitung nach einem der vorhergehenden Ansprüche, **dadurch gekennzeichnet, dass** die Zubereitung Natriumstearylglutamat in einer Konzentration von 0,05 bis 2 Gewichts-%, bezogen auf das Gesamtgewicht der Zubereitung, enthält.

8. Kosmetische Zubereitung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Glycyrrhetinsäure und/oder Licochalcon A enthält.

9. Kosmetische Zubereitung nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

10. Kosmetische Zubereitung nach einem der vorhergehenden Ansprüche, **dadurch gekennzeichnet, dass** die Zubereitung Phenoxyethanol enthält.

11. Verfahren zur Herstellung einer kosmetischen Zubereitung nach einem der vorhergehenden Ansprüche, welches **dadurch gekennzeichnet ist, dass** der Polyglyceryl Monoalkylester zunächst zusammen mit dem organischen UV-Filter aus der Gruppe 2,4,6-Tris-(biphenyl)-1,3,5-triazin; 2,2'-Methylen-bis-(6-(2H-benzotriazol-2-yl)-4-(1,1,3,3-tetramethylbutyl)-phenol) und/oder dem Hydroxyphenyl Benzophenon der Verbindung 1 zu einer wässrigen Vor-Dispersion vereinigt wird, die nach der Homogenisation in die abgekühlte fertige Emulsion welche die restlichen Bestandteile der Zubereitung enthält, unter Rühren zugegeben wird.

## Claims

1. Cosmetic preparation comprising a combination of
a) sodium stearylglutamate,
b) polyglyceryl monoalkyl ester,
wherein the preparation comprises 2,4,6-tris(biphenyl)-1,3,5-triazine, 2,2'-methylenebis(6-(2H-benzotriazol-2-yl)-4-(1,1,3,3-tetramethylbutyl)phenol) and/or the hydroxyphenyl benzophenone with the following structure (compound 1): 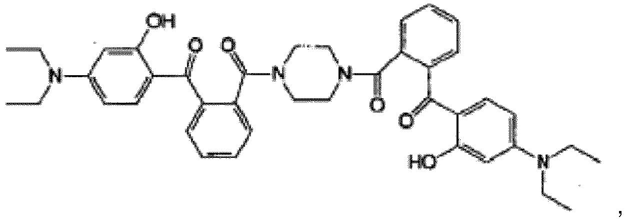 **characterized in that** the preparation comprises a polyglyceryl-10 monoalkyl ester as polyglyceryl monoalkyl ester.

2. Cosmetic preparation according to Claim 1, **characterized in that** the preparation polyglyceryl-10 stearate, polyglyceryl-10 laurate and/or polyglyceryl-10 caprate are used as polyglyceryl monoalkyl ester.

3. Cosmetic preparation according to one of the preceding claims, **characterized in that** the preparation comprises UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthylsalicylate; 2-ethylhexyl 2-hydroxybenzoate; 2-ethylhexyl 2-cyano-3,3-diphenylacrylate; dimethicodiethyl-benzalmalonate; 2-ethylhexyl 2-cyano-3,3-diphenylacrylate; 3-(4-(2,2-bis-ethoxycarbonylvinyl)phenoxy)propenyl)methoxysiloxane/dimethylsiloxane copolymer; dioctylbutylamidotriazone (INCI: Diethylhexyl-Butamidotriazone); 2,4-bis[5-1(dimethylpropyl)benzoxazole-2-yl-(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine; 4-dicyanomethylene-2,6-dimethyl-1,4-dihydropyridine N-(ethyloxysulphate ester salts), titanium dioxide; zinc oxide; polysiloxane copolymer with a random distribution according to the formula:

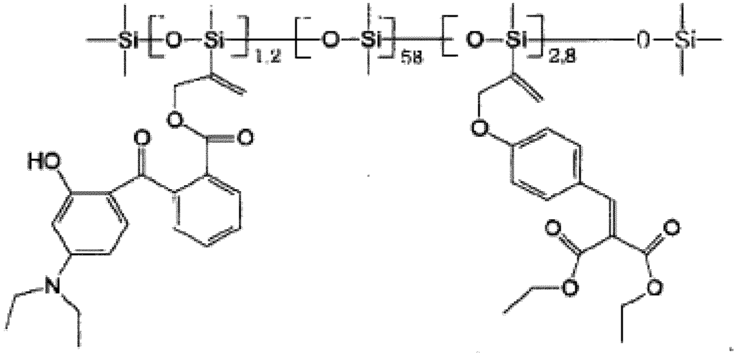

4. Cosmetic preparation according to one of the preceding claims, **characterized in that** the preparation comprises sucrose polystearate.

5. Cosmetic preparation according to one of the preceding claims, **characterized in that** the preparation comprises crosslinked polymer of vinylpyrrolidone and acrylic acid.

6. Cosmetic preparation according to one of the preceding claims, **characterized in that** the preparation comprises hydrogenated polyisobutene.

7. Cosmetic preparation according to one of the preceding claims, **characterized in that** the preparation comprises sodium stearylglutamate in a concentration of from 0.05 to 2% by weight, based on the total weight of the preparation.

8. Cosmetic preparation according to one of the preceding claims, **characterized in that** the preparation comprises one or more compounds selected from the group of the compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, glycerylglycose, (2-hydroxyethyl)urea, vitamin E and its derivatives, hyaluronic acid and/or its salts, glycyrrhetinic acid and/or licochalcone A.

9. Cosmetic preparation according to one of the preceding claims, **characterized in that** the preparation comprises one or more alkanediols from the group of the compounds 1,2-pentanediol, 1,2-hexanediol, 1,2-octanediol, 1,2-decanediol, 2-methyl-1,3-propanediol.

10. Cosmetic preparation according to one of the preceding claims, **characterized in that** the preparation comprises phenoxyethanol.

11. Process for producing a cosmetic preparation according to one of the preceding claims, which is **characterized in that** the polyglyceryl monoalkyl ester is combined firstly together with the organic UV filter from the group 2,4,6-tris(biphenyl)-1,3,5-triazine; 2,2'-methylenebis(6-(2H-benzotriazol-2-yl)-4-(1,1,3,3-tetramethylbutyl)phenol) and/or the hydroxyphenyl benzophenone of compound 1 to give an aqueous predispersion which, after homogenization, is added to the cooled finished emulsion which comprises the remaining constituents of the preparation, with stirring.

## Revendications

1. Composition cosmétique contenant une combinaison de
a) stéarylglutamate de sodium,
b) monoalkylester de polyglycéryle,
la composition contenant 2,4,6-tris-(biphényl)-1,3,5-triazine 2,2'-méthylène-bis-(6-(2H-benzotriazol-2-yl)-4-(1,1,3,3-tétraméthylbutyl)-phénol) et/ou l'hydroxyphénylbenzophénone présentant la structure suivante (composé 1) : 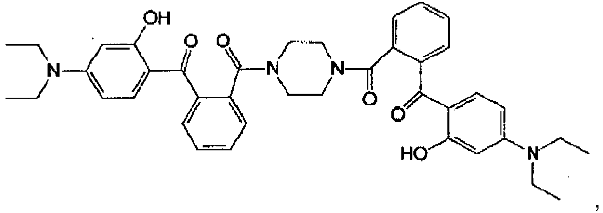 **caractérisée en ce que** la composition contient un monoalkylester de polyglycéryl-10 en tant que monoalkylester de polyglycéryle.

2. Composition cosmétique selon la revendication 1, **caractérisée en ce que** la composition sont utilisés le stéarate de polyglycéryl-10, le laurate de polyglycéryl-10 et/ou le caprate de polyglycéryl-10 en tant que monoalkylester de polyglycéryle.

3. Composition cosmétique selon l'une quelconque des revendications précédentes, **caractérisée en ce que** la composition contient un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle; benzalmalonate de diméthicodiéthyle ; 2-cyano-3,3-diphénylacrylate de 2-éthylhexyle ; copolymère de 3-(4-(2,2-bis(éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1-(diméthylpropyl)benzoxazol-2-yl-(4-phényl)-imino]-6-(2-éthylhexyl)-imino-1,3,5-triazine (présentant le n° CAS 288254-16-0) ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine) (INCI : Ethylhexyl Triazone) ; 2,4-bis-{[4-(2-éthylhexyloxy)-2-hydroxy]-phényl}-6-(4-méthoxyphényl)-1,3,5-triazine ; sels de l'ester N-(éthyloxysulfate) de la 4-dicyanométhylène-2,6-diméthyl-1,4-dihydropyridine, dioxyde de titane ; oxyde de zinc ; copolymère de polysiloxane présentant une distribution statistique selon la formule :

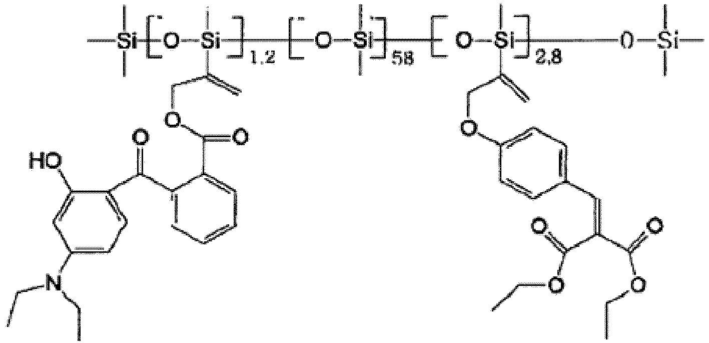

4. Composition cosmétique selon l'une quelconque des revendications précédentes, **caractérisée en ce que** la composition contient du polystéarate de saccharose.

5. Composition cosmétique selon l'une quelconque des revendications précédentes, **caractérisée en ce que** la composition contient un polymère réticulé de vinylpyrrolidone et d'acide acrylique.

6. Composition cosmétique selon l'une quelconque des revendications précédentes, **caractérisée en ce que** la composition contient du polyisobutène hydrogéné.

7. Composition cosmétique selon l'une quelconque des revendications précédentes, **caractérisée en ce que** la composition contient du stéarylglutamate de sodium en une concentration de 0,05 à 2% en poids par rapport au poids total de la composition.

8. Composition cosmétique selon l'une quelconque des revendications précédentes, **caractérisée en ce que** la composition contient un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels ou synthétiques, flavonoïdes, créatine, créatinine, taurine, β-alanine, acétate de tocophéryle, dihydroxyacétone ; acide 8-hexadécène-1,16-dicarboxylique, glycérylglycose, (2-hydroxyéthyl)urée, vitamine E ou, selon le cas ses dérivés, acide hyaluronique et/ou ses sels, acide glycyrrhétinique et/ou licochalcone A.

9. Composition cosmétique selon l'une des revendications précédentes, **caractérisée en ce que** la composition contient un ou plusieurs alcanediol(s) choisi(s) dans le groupe des composés 1,2-pentanediol, 1,2-hexanediol, 1,2-octanediol, 1,2-décanediol, 2-méthyl-1,3-propanediol.

10. Composition cosmétique selon l'une des revendications précédentes, **caractérisée en ce que** la composition contient du phéynoxyéthanol.

11. Procédé pour la préparation d'une composition cosmétique selon l'une quelconque des revendications précédentes, qui est **caractérisée en ce que** le monoalkylester de polyglycéryle est d'abord rassemblé, ensemble avec le filtre UV organique du groupe 2,4,6-tris-(biphényl)-1,3,5-triazine ; 2,2'-méthylènebis-(6-(2H-benzotriazol-2-yl)-4-(1,1,3,3-tétraméthylbutyl)-phénol) et/ou l'hydroxyphénylbenzophénone du composé 1, en une prédispersion aqueuse, qui est ajoutée, après homogénéisation, sous agitation, dans l'émulsion finie refroidie qui contient les autres constituants de la composition.
